# EUROPEAN PATENT SPECIFICATION

(11) **EP 3 401 667 B1**
(45) Date of publication and mention of the grant of the patent: **25.09.2019**
(21) Application number: 17198138.4
(22) Date of filing: 24.10.2017
(51) Int. Cl.: G01N 21/3563, G01N 33/02, G01N 21/359

(54) **METHOD AND APPARATUS FOR QUALITY EVALUATION OF PEANUTS SUITABLE FOR SNACK PEANUT PROCESSING**
VERFAHREN UND VORRICHTUNG ZUR QUALITÄTSBEURTEILUNG VON ERDNÜSSEN ZUR SNACK-ERDNUSSVERARBEITUNG
MÉTHODE ET APPAREIL D'ÉVALUATION DE LA QUALITÉ D'ARACHIDES CONVENANT POUR LE TRAITEMENT D'ARACHIDES DE COLLATION

(30) Priority: 09.05.2017 CN 201710322742
(43) Date of publication of application: 14.11.2018
(73) Proprietor: Institute of Food Science and Technology, Chinese Academy of Agricultural Sciences, 100193 Peking (CN)
(72) Inventor: HU, Hui, Beijing 100193 (CN); LIU, Li, Beijing 100193 (CN); LIU, Hongzhi, Beijing 100193 (CN); SHENG, Xiaojing, Beijing 100193 (CN); SHI, Aimin, Beijing 100193 (CN); WANG, Qiang, Beijing 100193 (CN)
(74) Representative: Geskes, Christoph

(56) References cited:
- CN-A- 106 053 742
- CN-B- 102 854 291
- SENGER ELISA ET AL: "Chuta (edibleJatropha curcasL.), the newcomer among underutilized crops: a rich source of vegetable oil and protein for human consumption", EUROPEAN FOOD RESEARCH AND TECHNOLOGY, SPRINGER BERLIN HEIDELBERG, BERLIN/HEIDELBERG, vol. 243, no. 6, 4 November 2016 (2016-11-04), pages 987-997, XP036223392, ISSN: 1438-2377, DOI: 10.1007/S00217-016-2814-X [retrieved on 2016-11-04]

## Description

### Technical Field

The present invention relates to the field of agricultural product processing technology, and particularly to a method and apparatus for quality evaluation of the peanuts suitable for snack peanut processing.

### Background

Peanut, which belongs to leguminosae, is originated from the tropical and subtropical regions of South America. Peanut is an important oil and protein raw material. The variety of peanut has a great impact on its quality. Since peanuts are mainly used for food processing but not used for oil expression, only the peanuts of certain varieties are preferred. In addition, with the development of global economy, the requirements for the hygiene, physicochemical and nutritive indexes of imported peanuts become more and more strict. In the past, the indexes of the size, color, water content, etc. of the peanuts were concerned only. But the requirements for the nutritive and physicochemical indexes of aflatoxin content, oleic acid and linoleic acid ratio, etc. are becoming stricter nowadays, which restricts the development of the peanut industry. Researches indicate that the peanuts of different varieties vary significantly in quality. Therefore, it is an important development direction of the peanut industry to further study the quality characteristics of different peanut varieties, and select the suitable peanut varieties.

At present, when evaluating the quality of the products, related hygiene index, physicochemical index and nutritive index are taken into account; and statistical processes such as analyzing main components are employed. Regression analysis from monitoring main components, which is often used in selecting near-infrared spectral wavelength, selecting environmental pollution indexes and studying disease factors, is widely used in modern agricultural science and related subjects. The methods above indeed play a role of reflecting problems "few but precisely". However, the current evaluating methods on whether the peanut is suitable for snack peanut processing usually need to collect indexes of various aspects of the peanut so as to give evaluation. The evaluation process is complicated.

During implementing the embodiments of the present invention, the applicant found that the current evaluating methods on whether the peanuts are suitable for snack peanut processing usually need to collect indexes of various aspects of the peanut so as to give evaluation. The evaluation process is complicated.

CN 102 854 291 B discloses a quality determination of peanuts suitable for peanut oil processing and an evaluation method thereof. CN 106 053 742 A relates to a quality determination method of peanuts for peanut sprouts and an evaluation method thereof.

### Summary

In the first aspect, the embodiments of the present disclosure provide a method for quality evaluation of the peanuts suitable for snack peanut processing, comprising:
obtaining the mass of the skin-removed peanuts to be measured, and obtaining the weight per hundred kernels of the peanuts, the water content of the peanuts, and the ratio of the oleic acid and linoleic acid contained in the peanuts;
calculating the quality evaluation factor associated with the peanuts, according to formula *Y* = 30.287+9.995×*a*+0.133×*b*-0.686×*c*; wherein *Y* is the quality evaluation factor associated with the peanuts, *a* is the ratio of the oleic acid and linoleic acid contained in the peanuts, b is the weight per hundred kernels of the peanuts, and c is the water content of the peanuts; and
evaluating the suitability of using the peanuts for snack peanut processing, according to the quality evaluation factor.

Alternatively, evaluating the suitability of using the peanuts for snack peanut processing, according to the quality evaluation factor, comprising:
if *Y*≤50, the peanuts are evaluated as unsuitable for snack peanut processing, otherwise, the peanuts are evaluated as suitable for snack peanut processing.

Alternatively, if the peanuts is suitable for snack peanut processing, the method further comprising:
if *Y≥*52*,* the suitability of using the peanuts for snack peanut processing is suitable; and
if 52≥*Y*≥50, the suitability of using the peanuts for snack peanut processing is basically suitable.

Alternatively, obtaining the water content of the peanuts, and the ratio of the oleic acid and linoleic acid contained in the peanuts, comprising:
heating the peanuts for a preset period of time, calculating the difference value between the mass of the peanuts before heating and the mass of the peanuts after heating, the difference value is the water content of the peanuts;
removing the skin from the peanuts, detecting the oleic acid and linoleic acid contained in the peanuts with near infrared analyzer, and calculating the ratio of the oleic acid and linoleic acid contained in the peanuts.

In the second aspect, the present invention provides an apparatus for quality evaluation of the peanut suitable for snack peanut processing, comprising:
an obtaining module, which is used for obtaining the mass of the skin-removed peanuts to be measured, and obtaining the weight per hundred kernels of the peanuts, the water content of the peanuts, and the ratio of the oleic acid and linoleic acid contained in the peanuts;
a calculating module, which is used for calculating the quality evaluation factor associated with the peanuts, according to to formula *Y* = 30.287+9.995×*a*+0.133×*b-*0.686×*c*; wherein *Y* is the quality evaluation factor associated with the peanuts, *a* is the ratio of the oleic acid and linoleic acid contained in the peanuts, *b* is the weight per hundred kernels of the peanuts, and *c* is the water content of the peanuts;
an evaluating module, which is used for evaluating the suitability of using the peanuts for snack peanut processing, according to the quality evaluation factor.

Alternatively, the evaluating module is further used that if *Y*≤50, the peanuts are unsuitable for snack peanut processing;
if *Y≥*52*,* the suitability of using the peanuts for snack peanut processing is suitable;
if 52≥*Y*≥50, the suitability of using the peanuts for snack peanut processing is basically suitable.

In the third aspect, the present invention provides an electronic device, comprising:
one or more processor; and
one or more memory communicated and connected to the processor, wherein:
   program instructions executable by the processor are stored in the memory, the processor is able to perform the methods above by calling the program instructions.

In the fourth aspect, the present invention provides a non-transient computer readable storage medium, the non-transient computer readable storage medium stores computer instructions enabling the computer to perform the methods above.

The embodiments of the present invention provide a method and apparatus for quality evaluation of the peanut suitable for snack peanut processing. The method could evaluate the quality of the peanuts and judge whether the peanuts are suitable for snack peanut processing, only by measuring the weight per hundred kernels, the water content and the oleic acid-linoleic acid ratio of the peanuts. The method is fast and convenient, and brings no damage to peanut kernels. On the other hand, it could also make a fast evaluation on the suitability of using the peanut for snack peanut processing with clustering analysis, according to the calculated quality evaluation factor of the peanut.

### Brief Description of the Drawings

In order to more clearly illustrate the embodiments of the present invention or the technical solutions in the prior art, the drawings which are to be used in the description of the embodiments or the prior art, will be briefly described hereinafter. It is obvious that the drawings in the following description are some embodiments of the present invention. For those skilled in the art, other drawings could also be obtained according to these drawings without paying creative work.
Figure 1 is a schematic flow chart of the method for quality evaluation of the peanut suitable for snack peanut processing provided by an embodiment of the present invention;
Figure 2 is structure block diagram of the apparatus for quality evaluation of the peanut suitable for snack peanut processing provided by an embodiment of the present invention;
Figure 3 is a physical structure block diagram of the electronic device provided by an embodiment of the present invention.

### Detailed Description

In order to make the objectives, technical solutions and merits of the present invention more clear, the technical solutions in the embodiments of the present disclosure will be described clearly and completely hereinafter with reference to the accompanying drawings for the embodiments of the present disclosure. It is obvious that the described embodiments are not all but only part of the embodiments of the present disclosure. Based on the embodiments in the present disclosure, all other embodiments obtained by those of ordinary skills in the art without inventive work are within the scope of the present disclosure.

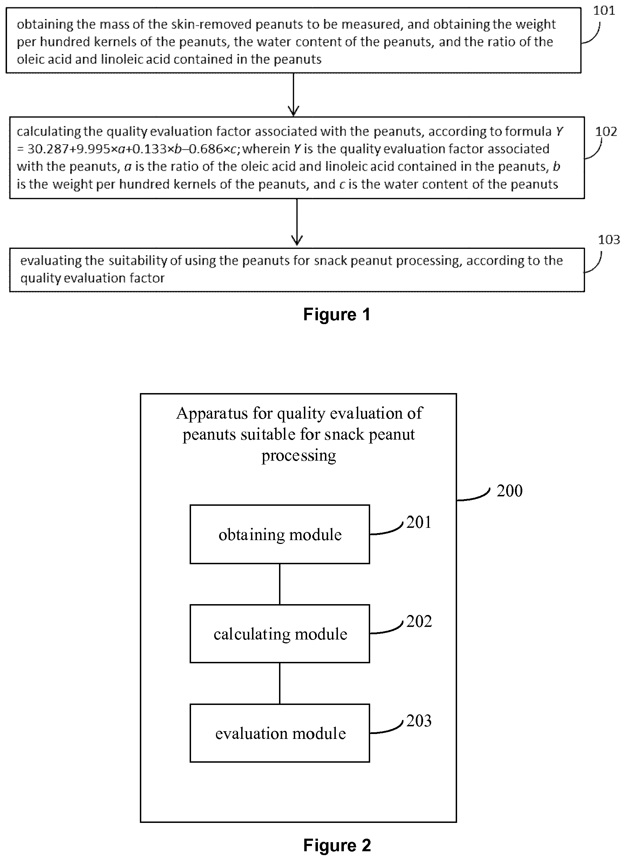

Figure 1 is a schematic flow chart of the method for quality evaluation of the peanut suitable for snack peanut processing provided by the embodiment. Refer to Figure 1, the method comprises:
101: obtaining the mass of the skin-removed peanuts to be measured, and obtaining the weight per hundred kernels of the peanuts, the water content of the peanuts, and the ratio of the oleic acid and linoleic acid contained in the peanuts;
102: calculating the quality evaluation factor associated with the peanuts, according to formula *Y* = 30.287+9.995×*a*+0.133×*b*-0.686×*c*; wherein *Y* is the quality evaluation factor associated with the peanuts, *a* is the ratio of the oleic acid and linoleic acid contained in the peanuts, *b* is the weight per hundred kernels of the peanuts, and *c* is the water content of the peanuts; and
103: evaluating the suitability of using the peanuts for snack peanut processing, according to the quality evaluation factor.

It should be noted that, the snack peanut refers to the peanut with certain tastes, which is processed from peanut and other materials, such as flour and seasonings. Usually, the peanut used for snack peanut processing is of relatively good quality, for example, the kernel is full; and the unsound kernel content is low, etc. The present embodiment provides a method for quality evaluation of the peanut suitable for snack peanut processing, which can evaluate the quality of the peanuts and judge whether the peanuts is suitable for snack peanut processing, only by measuring the weight per hundred kernels, the water content and the oleic acid-linoleic acid ratio of the peanuts. The method is fast and convenient, brings no damage to peanut kernels. On the other hand, it could also make a fast evaluation on the suitability of using the peanut for snack peanut processing with clustering analysis, according to the calculated quality evaluation factor of the peanut.

It should be noted that according to formula *Y*=30.287+9.995×*a*+0.133×*b*-0.686×*c*, the person conducting the peanut quality testing could judge whether the peanuts are suitable for snack peanut processing, only by obtaining the weight per hundred kernels, the water content and the oleic acid-linoleic acid ratio of the peanuts. And the obtaining method of the weight per hundred kernels, the water content and the ratio of the oleic acid and linoleic acid of the peanuts is simple and easy, therefore it is a convenient and fast evaluation method which brings no damage to peanut kernel.

In addition, on the basis of each embodiment above, evaluating the suitability of using the peanuts for snack peanut processing, according to the quality evaluation factor, comprising:
if *Y*≤50, the peanuts are unsuitable for snack peanut processing, otherwise, the peanuts are suitable for snack peanut processing.

Further, on the basis of each embodiment above, if the peanuts are suitable for snack peanut processing, it further comprising:
if *Y≥*52*,* the suitability of using the peanuts for snack peanut processing is suitable; and
if 52≥*Y*≥50, the suitability of using the peanuts for snack peanut processing is basically suitable.

For example, it is to make a K-means clustering analysis for the calculated quality evaluation factors with SPSS software, arrange the quality evaluation factors in the order of size and divide the factors into suitable, basically suitable and unsuitable. It is to classify the value of the quality evaluation factors into suitable, basically suitable and unsuitable with K-means clustering analysis by using quality evaluation model of the suitability for processing snack peanut (i.e. the formula *Y*=30.287+9.995×*a*+0.133×*b*-0.686×*c*). In the process of snack peanut processing, it is to preferably select the peanuts with the suitability of suitable for snack peanut processing, then select the peanuts with the suitability of basically suitable for snack peanut processing, avoiding selecting the peanuts with the suitability of unsuitable for snack peanut processing.

In addition, on the basis of each embodiment above, obtaining the water content of the peanuts, and the ratio of the oleic acid and linoleic acid contained in the peanuts, comprising:
heating the peanuts for a preset length of time, calculating the difference value between the mass of the peanuts before heating and the mass of the peanuts after heating, the difference value is the water content of the peanuts;
removing red skin from the peanuts, detecting the oleic acid and linoleic acid contained in the peanuts with near infrared analyzer, and calculating the ratio of the oleic acid and linoleic acid contained in the peanuts.

As a specific embodiment, the evaluation model is established as follows:
The peanut used for snack peanut processing contains 7 main physicochemical indexes, which are water content, aflatoxin content, oleic acid/linoleic acid ratio, unsound kernel content, red skin remaining rate, half-kernel content and grading respectively.

First, a certain score is given according to these physicochemical indexes' influence on the peanut quality. The sum of the scores of each index is the total score of the quality of the peanut. Detailed evaluation indexes and scoring criteria are in Table I.

In Table I, the water content of the peanut is determined according to the conventional method that the mass difference is measured by heating. This index is given 15 score.

The unsound kernels of peanut include moldy kernel, broken kernel, insect-bite and rat-bite kernel etc. Under normal conditions, the percentage of unsound kernels in total is less than 3%. This index is given 15 score, and the corresponding score obtained is calculated according to formula: 15-unsound kernel content×5.

The aflatoxin in peanuts is usually accumulated on the red skin. When the peanut is skin-removed, the aflatoxin content thereof would decrease significantly. As the peanut for snack peanut processing, its requirement of the aflatoxin content in the kernel is relatively high, which is usually no higher than 5µg/kg. Therefore, the index of aflatoxin content is given 20 score, and the score obtained from the aflatoxin content is calculated according to the formula (20-aflatoxin×4). When the aflatoxin content in a peanut exceeds 5µg/kg, the score obtained is 0.

At present, the peanut kernels are divided into two categories, one of which is large peanut kernel, the other is small peanut kernel. Wherein, more large peanut kernels are used for snack peanut processing, accounting for more than 80% of the total. Therefore, when considering the grading score of the suitability for processing snack peanut, the skin-removed large kernel is the main factor, and the skin-removed small kernel is the supplementary factor. Currently, the grading of the skin-removed large kernel and small kernel is 25/29, 29/33, 35/39, 39/43, 41/46, 46/51 and 51/61 (kernel/ounce). After researching the peanut enterprises such as Shandong Jinsheng Cereals & Oils Group, Junan Zhongsheng Cereals, Oils & Foodstuffs Co., Ltd., and Shangdong Lvdi Foodstuffs Co., Ltd., it is found that the large peanut kernel graded 25/29 has the largest amount, followed by the one graded 35/39 which is further followed by the one graded 29/33, those left are very few. Therefore, the index is given 15 score. Meanwhile, according to the information obtained during the researches, each grading is given a certain score based on the processing volume.

Peanut importers pay more attention to oleic acid/linoleic acid ratio, while most of the peanut varieties in China have relatively low oleic acid/linoleic acid ratio, which has become a major factor restricting the peanut processing and international trade competitiveness of China. The index is give 10 score. Under normal conditions, the oleic acid/linoleic acid ratios of the peanut varieties in China are mostly higher than 0.8, lower than 2.0. Therefore, the score obtained is calculated according to formula 10×(oleic acid/linoleic acid-0.8).

Under normal conditions, the merchants should require the index not to be higher than 2%. Therefore the index is given 15 score, and the score obtained is calculated according to 15-skin remaining rate×7.5, 0 is obtained if exceeding 2%.

**Table I Evaluation Criteria of the Indexes of Peanut's Physicochemical Properties**

| Index | Sensory Evaluation Criteria | Score |
|---|---|---|
| Water (15) | 15-water content×3 | 0-15 |
| Unsound Kernel (15) | 15-unsound kernel×5 | 0-15 |
| Half-kernel (10) | 10-half-kernel ratio×0.5 | 0-10 |
| Aflatoxin (15) | 20-aflatoxin content×4 | 0-20 |
| | 25/29 | 15 |
| | 29/33 | 11 |
| | 35/39 | 13 |
| Grading (15) | 39/43 | 9 |
| | 41/46 | 9 |
| | 46/51 | 8 |
| | 51/61 | 5 |
| Oleic Acid/Linoleic Acid (10) | 10×(oleic acid/linoleic acid)-0.8 | 10 |
| Remaining Seed Coat (15) | 15-remaining seed coat rate*7.5 | 15 |
| Total (100) | | 100 |

The total score of the variety is the sum of the scores of the seven indexes above in Table I, which is used for correlation analysis.

Subsequently, it is to make a correlation analysis of the peanut quality and the seven physicochemical properties above, the details are in Table II. Table II includes eleven indexes, which are respectively the weight per hundred kernels, fat content, protein content, total AA content, required AA content, total sugar content, oleic acid/linoleic acid, water, unsound kernel, aflatoxin content and seed coat color. These eleven indexes are used as the evaluation indexes for evaluating the processing characteristics and quality of the peanut.

It can be seen from Table II that, in the correlation analysis the weight per hundred pods (r=0.350) and the weight per hundred kernels (r=0.396) are in a significantly positive correlation with the total score of peanut's quality; kernel numbers per kilogram (r=0.387) is in a significantly negative correlation with the total score of peanut. Unsound kernel rate (r=-0.325) and aflatoxin content (r=-0.257) are in positive correlation with the total score of peanut. That is, the weight per hundred kernels (r=0.354), kernel numbers per kilogram (r=0.280), oleic acid content (r=0.475), oleic acid/linoleic acid ratio (r=0.460) and aflatoxin content (r=0.163) are in significantly or extremely significantly positive correlation with the total score of the skin-removed peanut kernel. Kernel production rate (r=-0.165), fat content (r=-0.176), linoleic acid content (r=-0.460), water (r=-0.147) are in significantly or extremely significantly negative correlation with the total score of peanut. There are also certain correlations between each physicochemical index of peanut and the quality indexes of peanut. As shown in Table II.

**Table II Correlation Analysis between the Physicochemical Properties and the Quality of Peanut**

| | Total score | Water content | Unsound kernel ratio | Half-kernel rate | Aflatoxin content | Specification score | Oleic acid/linoleic acid | Red skin remained |
|---|---|---|---|---|---|---|---|---|
| Weight per hundred kernels | 0.354** | -0.048 | -0.045 | -0.043 | -0.092 | 0.809** | 0.024 | -0.079 |
| Kernel numbers per kilogram | -0.280** | -0.023 | 0.001 | -0.091 | 0.079 | -0.505** | -0.091 | -0.001 |
| Kernel numbers per kilogram | -0.053 | 0.000 | 0.06 | 0.056 | 0.037 | -0.223** | -0.038 | 0.089 |
| Kernel production rate | -0.165* | -0.170* | -0.13 | -0.095 | -0.096 | 0.06 | -0.063 | -0.129 |
| Fat content | -0.176* | -0.038 | 0.033 | -0.051 | -0.051 | -0.132 | -0.105 | -0.088 |
| Total AA content | -0.075 | -0.081 | 0.111 | 0.025 | 0.018 | -0.239** | -0.043 | 0.128 |
| Required AA content | 0.001 | -0.009 | 0.095 | -0.108 | 0.071 | -0.256** | -0.007 | 0.133 |
| Protein content | -0.091 | -0.039 | 0.024 | -0.05 | -0.017 | -0.014 | -0.272** | 0.044 |
| Protein extraction rate | 0.083 | 0.057 | 0.084 | 0.079 | 0.077 | -0.072 | 0.021 | 0.095 |
| Nitrogen soluble index | 0.11 | 0.104 | 0.059 | 0.01 | 0.02 | -0.039 | 0.096 | 0.073 |
| Total sugar | -0.098 | -0.104 | 0.061 | 0.017 | -0.064 | -0.022 | -0.072 | -0.099 |
| Polysaccharide | 0.005 | 0.006 | 0.078 | -0.068 | -0.155* | 0.089 | -0.006 | 0.000 |
| Ash content | -0.124 | -0.06 | -0.128 | -0.124 | -0.14 | 0.001 | -0.189* | 0.113 |
| Oleic acid | 0.475** | -0.053 | -0.001 | 0.103 | 0.033 | 0.125 | 0.894** | -0.061 |
| Linoleic acid | -0.460** | 0.000 | 0.025 | -0.004 | 0.036 | -0.048 | -0.921** | -0.028 |
| Oleic acid/linoleic acid | 0.517** | -0.020 | -0.023 | 0.054 | 0.005 | 0.099 | 1.000** | -0.021 |
| Water | -0.147* | -0.192* | -0.02 | -0.092 | -0.086 | 0.137 | -0.178* | -0.121 |
| Unsound kernel | 0.103 | 0.205** | -0.011 | 0.095 | 0.082 | -0.137 | 0.15 | 0.104 |
| Aflatoxin | 0.163* | 0.153* | 0.067 | -0.062 | -0.06 | 0.103 | -0.018 | 0.232** |

Eleven indexes which are the weight per hundred kernels, fat content, protein content, total AA content, required AA content, total sugar content, oleic acid/linoleic acid, water, unsound kernel, aflatoxin content and seed coat color in 169 peanuts varieties are independent variables, the total score of the skin-removed peanut is dependent variable. It is found after stepwise regression analysis that three indexes which are the weight per hundred kernels, oleic acid/linoleic acid ratio and water content are incorporated, and the regression equation formed eventually is: *Y* = 30.287+9.995×*a*+0.133×*b*-0.686×*c*, wherein, *Y* is the quality evaluation factor associated with the peanuts, *a* is the ratio of the oleic acid and linoleic acid contained in the peanuts, *b* is the hundred-kernel weight of the peanuts, and *c* is the water content of the peanuts.

It can be seen from the regression coefficient Table III that, the significant levels of the weight per hundred kernels, oleic acid/linoleic acid ratio and water content are less than 0.05, which indicates that the influential effect of the independent variables and dependent variables is significant, and there is a significant linear regression relation.

**Table III Coefficients Table of the Peanut Quality Regression Model**

| | Non-standard coefficient | | Standard coefficient | | |
|---|---|---|---|---|---|
| Model | Regression coefficient | Standard deviation | Trail | t | Sig. |
| Constant | 30.287 | 2.523 | | 12.006 | 0.000 |
| Weight per hundred kernels | 9.995 | 1.37 | 0.485 | 7.296 | 0.000 |
| Oleic acid/linoleic acid ratio | 0.133 | 0.022 | 0.409 | 5.935 | 0.000 |
| Aflatoxin content | -0.686 | 0.294 | -0.162 | -2.329 | 0.021 |

The effectiveness of the quality regression model of skin-removed peanut kernel is judged through F test. Therefore, a variance analysis is conducted on the model. It is obtained from the variance analysis (refer to Table IV) that, the statistical magnitude of the regression equation is F=32.75, significance test result Sig=0.000, which means a significant linear relation of the multivariable regression equation. Thus, it is effective for the established multivariable linear regression model to speculate the total score of the quality of the skin-removed peanut kernel.

**Table IV Variance Analysis Table**

| Model | Quadratic sum | Degree of freedom | Average variance | F | Sig. |
|---|---|---|---|---|---|
| Regression | 1664.229 | 3 | 554.743 | 32.75 | .000 |
| Residual | 2269.787 | 134 | 16.939 | | |
| Total | 3934.016 | 137 | | | |

In order to verify the model above, except for the 169 peanut varieties used in the peanut quality regression model, another 10 peanut varieties are selected, which are HQ 010, Huayu 21, Quanhua 464, Yueyou 551, HY 256, Heyou 4, Jihua 2, Huayu 17, Guihua 17 and Qinglan 2. The property analysis results of these 10 peanut varieties are shown in Table 2-5. Three property measure results of these 10 peanut varieties are substituted into the snack peanut quality regression equation prediction model *Y* = 30.287+9.995×*a*+0.133×*b*-0.686×*c*, wherein, *Y* is the quality evaluation factor associated with the peanuts, *a* is the ratio of the oleic acid and linoleic acid contained in the peanuts, *b* is the weight per hundred kernels of the peanuts, and *c* is the water content of the peanuts.

A comparison validation is made between the obtained prediction values and experiment values. The result shows that the relative error between the total score of the skin-removed peanut kernel and the model prediction value is only around 10%, the correlation coefficient is 0.748, the significance is 0.013, which indicates that there is an extremely significant correlation at the level of 0.01 (as shown in Table V, Table VI and Table VII).

**Table V Prediction Score of 10 Peanut Varieties**

| Peanut variety | Weight per hundred kernels/g | o/s | Aflatoxin content | Prediction score |
|---|---|---|---|---|
| HQ 010 | 59.50 | 1.27 | 0.67 | 42.14 |
| Huayu 21 | 72.90 | 1.11 | 0.77 | 42.26 |
| Quanhua 464 | 68.05 | 1.34 | 0.26 | 44.11 |
| Yueyou 551 | 56.90 | 0.96 | 0.84 | 39.80 |
| HY 256 | 53.05 | 1.16 | 1.33 | 39.70 |
| Heyou 4 | 68.60 | 1.05 | 0.48 | 42.09 |
| Jihua 2 | 74.10 | 1.30 | 1.23 | 42.58 |
| Huayu 17 | 92.30 | 1.14 | 1.35 | 43.09 |
| Guihua 17 | 55.00 | 1.16 | 0.30 | 41.80 |
| Qinglan 2 (small) | 52.90 | 1.30 | 0.83 | 41.43 |

**Table VI Actual Score of the 10 Peanut Varieties**

| Peanut variety | Water of skin-removed kernel | Water score | Unsound kernel ratio | Unsound kernel score | Half-kernel score ratio | Half-kernel score | Aflatoxin content | Aflatoxin content score | Specification | Specification score | o/s | os score | Seed coat remained | Score of the remains | Total score |
|---|---|---|---|---|---|---|---|---|---|---|---|---|---|---|---|
| HQ 010 | 3.65 | 4.04 | 1.01 | 9.97 | 9.81 | 5.10 | 2.68 | 9.27 | 47.65 | 8 | 1.27 | 4.69 | 1.01 | 7.46 | 48.53 |
| Huayu 21 | 3.51 | 4.47 | 1.38 | 8.11 | 10.10 | 4.95 | 2.51 | 9.95 | 38.89 | 13 | 1.11 | 3.07 | 0.63 | 10.28 | 53.82 |
| Quanhua 464 | 3.48 | 4.56 | 1.17 | 9.16 | 11.47 | 4.27 | 2.25 | 10.99 | 41.66 | 9 | 1.34 | 5.41 | 0.58 | 10.68 | 54.08 |
| Yueyou 551 | 4.00 | 3.00 | 0.91 | 10.44 | 11.07 | 4.47 | 3.46 | 6.18 | 49.82 | 8 | 0.96 | 1.56 | 0.76 | 9.28 | 42.91 |
| HY 256 | 4.08 | 2.77 | 1.14 | 9.32 | 9.71 | 5.15 | 2.33 | 10.69 | 53.44 | 5 | 1.16 | 3.64 | 1.15 | 6.40 | 42.96 |
| Heyou 4 | 4.10 | 2.69 | 0.70 | 11.51 | 10.12 | 4.94 | 3.06 | 7.74 | 41.33 | 9 | 1.05 | 2.50 | 1.02 | 7.33 | 45.71 |
| Jihua 2 | 3.99 | 3.02 | 0.96 | 10.20 | 10.75 | 4.63 | 2.72 | 9.11 | 38.26 | 13 | 1.30 | 4.99 | 1.00 | 7.49 | 52.44 |
| Huayu 17 | 3.83 | 3.50 | 0.66 | 11.68 | 10.19 | 4.90 | 2.78 | 8.90 | 30.72 | 11 | 1.14 | 3.40 | 0.88 | 8.40 | 51.78 |
| Guihua 17 | 4.11 | 2.67 | 1.23 | 8.86 | 9.70 | 5.15 | 3.42 | 6.30 | 51.55 | 5 | 1.16 | 3.56 | 0.89 | 8.34 | 39.89 |
| Qinglan 2 (small) | 3.65 | 4.04 | 0.54 | 12.32 | 11.48 | 4.26 | 3.36 | 6.55 | 53.59 | 5 | 1.30 | 5.04 | 1.08 | 6.94 | 44.15 |

**Table VII Verification Analysis of the Peanut Quality Prediction Model**

| | Prediction value | Total score | Relative error | Correlation coefficient | Significant level |
|---|---|---|---|---|---|
| HQ 010 | 42.14 | 48.53 | 4.52% | 0.748 | 0.013 |
| Huayu 21 | 42.26 | 53.82 | 8.17% | | |
| Quanhua 464 | 44.11 | 54.08 | 7.06% | | |
| Yueyou 551 | 39.80 | 42.91 | 2.20% | | |
| HY 256 | 39.70 | 42.96 | 2.30% | | |
| Heyou 4 | 42.09 | 45.71 | 2.56% | | |
| Jihua 2 | 42.58 | 52.44 | 6.97% | | |
| Huayu 17 | 43.09 | 51.78 | 6.14% | | |
| Guihua 17 | 41.80 | 39.89 | 1.35% | | |
| Qinglan 2 (small) | 41.43 | 44.15 | 1.92% | | |

This embodiment obtains 3 indexes associated with the model above by using 169 varieties, and verified the model (*Y* = 30.287+9.995×*a*+0.133×*b*-0.686×*c*) with 10 varieties, the correlation coefficient between the prediction value and actual value is 0.748, indicating that the prediction result of the equation is relatively greater. Applying the model could determine the evaluation indexes of peanut quality, and predict the quality of the unknown peanut, provide the basis for processing enterprises to select peanuts varieties suitable for processing.

*Y* = 30.287+9.995×*a*+0.133×*b*-0.686×*c*, wherein, *Y* is the quality evaluation factor associated with the peanuts, *a* is the ratio of the oleic acid and linoleic acid contained in the peanuts, *b* is the weight per hundred kernels of the peanuts, and *c* is the water content of the peanuts.

It can be seen from Table VIII that, the weight per hundred kernels, aflatoxin content (the ratio of the oleic acid and linoleic acid), water content are in extremely significant correlation with the total score, indicating that the three indexes are the significant factors influencing peanut quality factor.

The significances of the correlation coefficients between the weight per hundred kernels, oleic acid/linoleic acid ratio and aflatoxin content are more than 0.5, indicating that the three indexes are relatively independent from each other, could individually reflect the relation between the quality of the skin-removed peanut kernel.

**Table VIII Correlation Analysis between the Three Indexes and the Total score**

| | | Weight per hundred kernels | Oleic acid/linoleic acid ratio | Aflatoxin content | Total score |
|---|---|---|---|---|---|
| Weight per hundred kernels | Correlation coefficient | 1 | 0.021 | 0.048 | .354** |
| | Significance | | 0.792 | 0.526 | 0 |
| Oleic acid/linoleic acid ratio | Correlation coefficient | 0.021 | 1 | 0.02 | .517** |
| | Significance | 0.792 | | 0.802 | 0 |
| Water content | Correlation coefficient | 0.048 | 0.02 | 1 | -.296** |
| | Significance | 0.526 | 0.802 | | 0 |
| Total score | Correlation coefficient | .354** | .517** | -.296** | 1 |
| | Significance | 1 | 0.021 | 0.048 | .354** |

For example, by applying the model *Y* = 30.287+9.995×*a*+0.133×*b*-0.686×*c*, wherein, *Y* is the quality evaluation factor associated with the peanuts, *a* is the ratio of the oleic acid and linoleic acid contained in the peanuts, *b* is the weight per hundred kernels of the peanuts, *c* is the water content of the peanuts, the suitability for snack peanut processing of peanuts of 179 varieties are evaluated, as shown in Table IX.

**Table IX Table of the Suitability for Snack Peanut Processing of Peanut Varieties**

| Clustering order | Variety number | Variety name |
|---|---|---|
| Suitable | 59 (52.23±3.95) | Huayu 8, Huayu 16, Huayu 25, Huayu 31, Luhua 9, Fenghua 1, Variety 060, Baisha 101, Shanhua 7, Shanhua 9, Haihua 1, Haiyu 6, Bianhua 3, Honghua 1, Xuhua 14, Hongguan, 5-color peanut, Zhongyu 1 black peanut, 034-256-1, Ji 9814, Zhennong 7, Yuanza 9102, 818, Longhua 243, Shanyou 250, Yueyou 55, Heyou 11, Sishuizhanyang, Hua 93, Xintaizhanyang, Linqingyiwofeng, Linhua 2, Qixiabankangpi, Wendengbanman, Qixialaobaoji, Qixiadaliman, Jimopaman, Qingdaopaman, Wenshangmansheng, Guihua 35, Luhua 12, Luhua 10, Qingdaomanshengdali, Qingdaoliman, Huayu 36, Xiangxiang, Mengyinbanzhanmandahuasheng, Zhongnong 8, HO616, Huayu 21, HL008, HQ327, Quanhua 464, Minhua 10, Zhufeng 1, Hua 37, 8159, Huayu 17, Qinglan 2 (large) |
| Basically suitable | 49 (50.82±5.13) | Huayu 19, Hauyu 22, Huayu 28, luhua 11, Luhua 14, Fenghua 3, Fenghua 5, Lufeng 2, 9616, Baisha 1016, Zhongnong 108, Qinglan 8, Shuangji 2, Yuanhua 8, Xuhua 13, Huaguanwang, 780-15, 365-1, L03-329-336, L03-601-604, Yueyou 52, Yuhua 15, Yuhua 9326, Minhua 9, Yuanza 9307, Kunyudalidun, Mupingdunhuasheng, Juyetuoyang, Laixiqilidun, Muzhukuangbanman, Penglaizaohuasheng, Qixiabuluoye, Laiyangdunhuasheng, Laixiliman, Ningyangdaliman, Dingtaobanmanyanghuasheng, Hua 55, Linhua 1, Laiyanglaobaoji, Pingdupaman, Hua 67, Xixuan 21, Zhongkaihua 4, Huayu 33, Shuangji 22, HE006, HY005, Jihua 2, 8186 |
| Unsuitable | 71 (45.00±3.80) | Huayu 20, Huayu 23, Luhua 15, Fenghua 4, Fenghua 6, Zhonghua 4, Zhonghua 8, Xuhua 5, Xuhua 15, Kainongbai 2 white peanut, Fuhua 1 Silihong, 8130, Fua 17, Xianghua 509-77, L03-257-294, Yueyou 7, Yueyou 14, Yueyou 20, Yueyou 39, Yueyou 40, Yueyou 45, Yueyou 86, Zhanhua 82 (oil), Puhua 23, Zhenzhuhong (zhenzhuhong 1), Yuhua 9327, Quanhua 551, Guihua 771, Yexianyiwohou, Pingdulijing, Mengyinbanzhanyangdahuasheng, Laiyangbanman, Haiyangpaman, Fushanruanpiman, Zhaoyuanbanman, Yishuidaliman, Laiwupaman, Taianxiaojinguo, Juanchenglayang, Xiaopuyang, Guihua 95, Guihua 166, Luhua 10, Tianfu 4, Laiyangxuefangbanman, Huayu 25, Huayu 32, Luhua 2, Yueyou 92, HQ010, Yueyou 551, HY256, Heyou 4, Guihua 17, Qinglan 2 (small), Zhonghua 15, 600-6, 606, Nongda 108, Rihua 2, L-6215, Zhenzhuhei, Zhongkaihua 9, Dongpinglimanhuasheng, Mupingyiwohou, Qufudoupeng, Qixiabuluoyelaobaoji, Zhaoyuanbanman, Pingdudalidun, Cangshansiguozi |

The method for quality evaluation of the peanut suitable for snack peanut processing provided by the embodiment, measures the weight per hundred kernels, oleic acid/linoleic acid ratio, water content of the peanuts; substitutes each measured values into *Y* = 30.287+9.995×*a*+0.133×*b*-0.686×*c* (*Y* is the quality evaluation factor associated with the peanuts, *a* is the ratio of the oleic acid and linoleic acid contained in the peanuts, *b* is the weight per hundred kernels of the peanuts, *c* is the water content of the peanuts), and thus obtains the quality evaluation factor of the peanuts. It is to make a K-means clustering analysis for the synthetical values of the qualities of snack peanuts with SPSS software, arranges them in the order of size and divide them into suitable, basically suitable and unsuitable. On the other hand, as shown in Figure 2, the embodiment provides a apparatus 200 for quality evaluation of the peanut suitable for snack peanut processing, which comprises: obtaining module 201, calculating module 202 and evaluating module 203, wherein,
the obtaining module 201 is used for obtaining the mass of the skin-removed peanuts, and obtaining the weight per hundred kernels of the peanuts, the water content of the peanuts, and the ratio of the oleic acid and linoleic acid contained in the peanuts;
the calculating module 202 is used for calculating the quality evaluation factor associated with the peanuts, according to the weight per hundred kernels, the water content and the ratio; and
the evaluating module 203 is used for evaluating the suitability of using the peanuts for snack peanut processing, according to the quality evaluation factor.

The apparatus 200 for quality evaluation of the peanut suitable for snack peanut processing provided by the embodiment, applies to the method for quality evaluation of the peanut suitable for snack peanut processing in the embodiments above, a repetitive description is omitted herein.

The apparatus for quality evaluation of the peanut suitable for snack peanut processing provided by the embodiment, could evaluate the quality of the peanuts and judge whether the peanuts are suitable for snack peanut processing, only by measuring the weight per hundred kernels, the water content and the oleic acid-linoleic acid ratio of the peanuts. The method is fast and convenient, brings no damage to peanut kernels. On the other hand, it could also make a fast evaluation on the suitability of using the peanut for snack peanut processing with clustering analysis, according to the calculated quality evaluation factor of the peanut.

In addition, on the basis of the embodiment above, the calculating module is also used for calculating the quality evaluation factor associated with the peanuts according to formula *Y* = 30.287+9.995×*a*+0.133×*b*-0.686×*c*;

Wherein *Y* is the quality evaluation factor associated with the peanuts, *a* is the ratio of the oleic acid and linoleic acid contained in the peanuts, *b* is the weight per hundred kernels of the peanuts, and *c* is the water content of the peanuts.

In addition, on the basis of each embodiment above, the evaluation module is also used that if *Y*≤50, the peanuts are unsuitable for snack peanut processing;
If *Y≥*52*,* the suitability of using the peanuts for snack peanut processing is suitable;
If 52≥*Y*≥50, the suitability of using the peanuts for snack peanut processing is basically suitable.

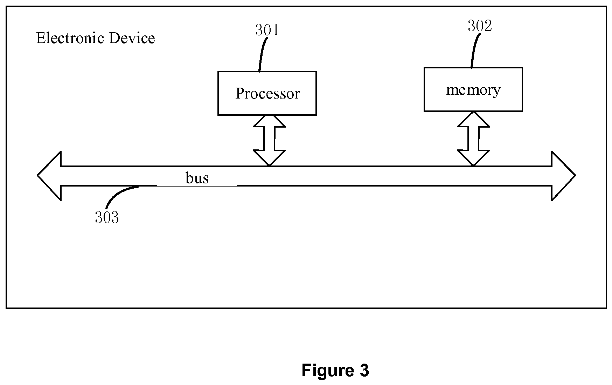

Figure 3 is a structure block diagram illustrating the electronic device according to the embodiment of the present invention.

Refer to Figure 3, the electronic device comprises: processor 301, memory 302 and bus 303;
Wherein, the processor 301 and memory 302 perform the communication with each other through the bus 303;
The processor 301 is used for calling the program instructions in the memory 302, so as to perform the methods provided by each method embodiment above. For example, the methods comprise: obtaining the mass of the skin-removed peanuts, and obtaining the weight per hundred kernels of the peanuts, the water content of the peanuts, and the ratio of the oleic acid and linoleic acid contained in the peanuts; calculating the quality evaluation factor associated with the peanuts, according to the weight per hundred kernels, the water content and the ratio; and evaluating the suitability of using the peanuts for snack peanut processing, according to the quality evaluation factor.

The embodiment provides a computer program product, the computer program product comprises computer programs stored on the non-transient computer readable storage medium, the computer program comprises program instructions, and a computer could perform the methods provided by each method embodiment above when the program instructions are performed by the computer. For example, the methods comprise: obtaining the mass of the skin-removed peanuts, and obtaining the weight per hundred kernels of the peanuts, the water content of the peanuts, and the ratio of the oleic acid and linoleic acid contained in the peanuts; calculating the quality evaluation factor associated with the peanuts, according to the weight per hundred kernels, the water content and the ratio; and evaluating the suitability of using the peanuts for snack peanut processing, according to the quality evaluation factor.

The embodiment provides a non-transient computer readable storage medium, the non-transient computer readable storage medium stores computer instructions which instruct the computer to perform the methods provided by each method embodiment above. For example, the methods comprise: obtaining the mass of the skin-removed peanut to be measured, and obtaining the weight per hundred kernels of the peanuts, the water content of the peanuts, and the ratio of the oleic acid and linoleic acid contained in the peanuts; calculating the quality evaluation factor associated with the peanuts, according to the weight per hundred kernels, the water content and the ratio; and evaluating the suitability of using the peanuts for snack peanut processing, according to the quality evaluation factor.

It will be understood by those of ordinary skills in the art that, all or part of the steps for implementing the method embodiments described above, may be accomplished by means of the hardware associated with program instructions. The aforementioned programs may be stored in a computer readable storage medium. When the programs are executed, the steps including those of the method embodiments described above are performed; and the aforementioned storage medium includes various mediums which could store program codes, such as ROM, RAM, disk or compact disk.

The embodiments such as the electronic devices described above are merely illustrative, wherein the units which are described as separate components, may be or may not be physically separate, and the components shown as units may be or may not be physical units, that is, could be located in one place, or could be distributed to multiple network elements. The objectives of the embodiment could be achieved by selecting part or all of the modules therein according to the actual needs. Those skilled in the art could understand and practice without paying creative work.

With the description of the implementing methods above, those skilled in the art could understand clearly that each implementing method may be realized by means of software with necessary general hardware platform, and of course may be realized by hardware. Based on this understanding, the essence of the above-mentioned technical solutions, or the parts thereof contributing to the existing technology, could be embodied in the form of software products. The computer software products could be stored in a computer readable storage medium, such as ROM/RAM, disk or compact disk, including several instructions for enabling a computer device (may be a personal computer, a server, or a network device) to perform the methods described by each embodiment or some parts of the embodiment.

It should be noted that: each of the embodiments above is only used for describing rather than limiting the present invention; although the present invention has been described in detail with reference to each foregoing embodiment, it should be understood by those of ordinary skills in the art that it is still possible to modify the technical solutions described in the foregoing embodiments, or to equivalently substitute some or all of the technical features therein; and these modifications or substitutions do not separate the essence of corresponding technical solutions from the scope of the technical solutions within each embodiment of the present invention.

## Claims

1. A method for quality evaluation of peanuts suitable for snack peanut processing, **characterized in that**, comprising:
obtaining the mass of the skin-removed peanuts to be measured, and obtaining the weight per hundred kernels of the peanuts, the water content of the peanuts, and the ratio of the oleic acid and linoleic acid contained in the peanuts;
calculating the quality evaluation factor associated with the peanuts according to formula *Y* = 30.287+9.995×*a*+0.133×*b*-0.686×*c*; wherein *Y* is the quality evaluation factor associated with the peanuts, *a* is the ratio of the oleic acid and linoleic acid contained in the peanuts, *b* is the weight per hundred kernels of the peanuts in grams, and *c* is the water content of the peanuts; and
evaluating the suitability of using the peanuts for snack peanut processing according to the quality evaluation factor.

2. The method according to claim 1, **characterized in that**, evaluating the suitability of using the peanuts for snack peanut processing according to the quality evaluation factor, comprising:
if *Y*≤50, the peanuts are unsuitable for snack peanut processing; otherwise, the peanuts are suitable for snack peanut processing.

3. The method according to claim 2, **characterized in that**, if the peanuts are suitable for snack peanut processing, further comprising:
if *Y≥*52*,* the suitability of using the peanuts for snack peanut processing are suitable; and
if 52≥*Y*≥50, the suitability of using the peanuts for snack peanut processing are basically suitable.

4. The method according to claim 1, **characterized in that**, obtaining the water content of the peanuts, and the ratio of the oleic acid and linoleic acid contained in the peanuts, comprising:
heating the peanuts for a preset period of time, calculating the difference value between the mass of the peanuts before heating and the mass of the peanuts after heating, the difference value is the water content of the peanuts; and
removing skin from the peanuts, detecting the oleic acid and linoleic acid contained in the peanuts with near infrared analyzer, and calculating the ratio of the oleic acid and linoleic acid contained in the peanuts.

5. An apparatus for quality evaluation of peanuts suitable for snack peanut processing, **characterized in that**, comprising:
an obtaining module, which is used for obtaining the mass of the skin-removed peanuts to be measured, and obtaining the weight per hundred kernels of the peanuts, the water content of the peanuts, and the ratio of the oleic acid and linoleic acid contained in the peanuts;
a calculating module, which is used for calculating the quality evaluation factor associated with the peanuts according to formula *Y* = 30.287+9.995×*a*+0.133×*b*-0.686×*c*;
wherein *Y* is the quality evaluation factor associated with the peanuts, *a* is the ratio of the oleic acid and linoleic acid contained in the peanuts, *b* is the weight per hundred kernels of the peanuts in grams, and *c* is the water content of the peanuts; and
an evaluating module, which is used for evaluating the suitability of using the peanuts for snack peanut processing, according to the quality evaluation factor.

6. The apparatus according to claim 5, **characterized in that**, the evaluating module is further used that if *Y*≤50, the peanuts are unsuitable for snack peanut processing;
if *Y≥*52*,* the suitability of using the peanuts for snack peanut processing are suitable; and if 52≥*Y*≥50, the suitability of using the peanuts for snack peanut processing are basically suitable.

7. A non-transient computer readable storage medium, **characterized in that**, the non-transient computer readable storage medium stores computer instructions enabling the computer to perform the methods as described by any one of claims 1 to 4.

## Patentansprüche

1. Verfahren zur Qualitätsbewertung von Erdnüssen für die Snack-Erdnussverarbeitung, **dadurch gekennzeichnet, dass** es umfasst:
Erhalten der zu messenden Masse an enthäuteten Erdnüssen, und Bestimmen des Gewichtes pro hundert Erdnusskerne, des Wassergehalts der Erdnüsse, und des Gehalts der in den Erdnüssen enthaltenen Ölsäure und Linolsäure;
Berechnen des den Erdnüssen zugeordneten Qualitätsbewertungsfaktors aus der Formel *Y* = 30,287+9,995×*a*+0,133×*b*-0,686×*c*; wobei *Y* der den Erdnüssen zugeordnete Qualitätsbewertungsfaktor, o der Gehalt an Ölsäure und Linolsäure in den Erdnüssen, b das Gewicht pro hundert Erdnusskerne in Gramm, und c der Wassergehalt der Erdnüsse ist; und
Bewerten anhand des Qualitätsbewertungsfaktors, ob die Erdnüsse für die Weiterverarbeitung zu Snack-Erdnüssen geeignet sind.

2. Verfahren gemäß Anspruch 1, **gekennzeichnet durch** das Bewerten anhand des Qualitätsbewertungsfaktors, ob die Erdnüsse für die Weiterverarbeitung zu Snack-Erdnüssen geeignet sind, umfassend:
wenn Y≤50, sind die Erdnüsse nicht für die Weiterverarbeitung zu Snack-Erdnüssen geeignet; anderenfalls sind sie geeignet für die Weiterverarbeitung zu Snack-Erdnüssen.

3. Verfahren gemäß Anspruch 1, **dadurch gekennzeichnet, dass**, wenn die Erdnüsse für die Weiterverarbeitung zu Snack-Erdnüssen geeignet sind, dieses umfasst:
wenn *Y≥*52*,* ist die Tauglichkeit der Verwendung der Erdnüsse für die Erdnuss-Snack-Verarbeitung gegeben; und wenn 52≥*Y*≥50, ist die Tauglichkeit der Verwendung der Erdnüsse für die Erdnuss-Snack-Verarbeitung grundsätzlich gegeben.

4. Verfahren gemäß Anspruch 1, **gekennzeichnet durch** das Bestimmen des Wassergehalts der Erdnüsse und der in den Erdnüssen enthaltenen Ölsäure und Linolsäure, umfassend:
Erhitzen der Erdnüsse für eine vorgegebene Zeitspanne, Errechnen des Differenzwertes zwischen der Erdnussmasse vor dem Erhitzen und der Erdnussmasse nach dem Erhitzen, wobei der Differenzwert der Wassergehalt der Erdnüsse ist; und
Entfernen der Haut von den Erdnüssen, Messen der in den Erdnüssen enthaltenen Ölsäure und Linolsäure mit einem Nah-Infrarot-Analysegerät, und Berechnen des in den Erdnüssen enthaltenen Gehalts an Ölsäure und Linolsäure.

5. Vorrichtung zur Qualitätsbewertung von für die Snack-Erdnussverarbeitung geeigneten Erdnüssen, **dadurch gekennzeichnet, dass** diese umfasst:
ein Berechnungsmodul zum Berechnen des den Erdnüssen zugeordneten Qualitätsbewertungsfaktors aus der Formel *Y* = 30,287+9,995×*a*+0,133×*b*-0,686×*c*; wobei *Y* der den Erdnüssen zugeordnete Qualitätsbewertungsfaktor, *a* der Gehalt an Ölsäure und Linolsäure in den Erdnüssen, *b* das Gewicht pro hundert Erdnusskerne in Gramm, und *c* der Wassergehalt der Erdnüsse ist; und
ein Auswertungsmodul zum Bewerten, ob die Erdnüsse für die Weiterverarbeitung zu Snack-Erdnüssen geeignet sind anhand des Qualitätsbewertungsfaktors.

6. Vorrichtung gemäß Anspruch 5, **dadurch gekennzeichnet, dass** das Auswertungsmodul weiterhin verwendet wird zum Bewerten, dass, wenn *Y*≤50, die Erdnüsse nicht zur Weiterverarbeitung zu Snack-Erdnüssen geeignet sind;
wenn *Y≥*52*,* die Tauglichkeit der Verwendung der Erdnüsse für die Erdnuss-Snack-Verarbeitung gegeben ist; und wenn 52≥*Y*≥50, die Tauglichkeit der Verwendung der Erdnüsse für die Erdnuss-Snack-Verarbeitung grundsätzlich gegeben ist.

7. Dauerhaftes, computerlesbares Speichermedium, **dadurch gekennzeichnet, dass** das dauerhafte computerlesbare Speichermedium Computeranweisungen speichert, die den Computer aktivieren, das in den Ansprüchen 1 bis 4 beschriebene Verfahren auszuführen.

## Revendications

1. Procédé pour l'évaluation de la qualité d'arachides convenant pour le traitement d'arachides de collation, **caractérisé en ce qu'**il comprend :
l'obtention de la masse d'arachides décortiquées à mesurer, et l'obtention du poids pour cent noyaux des arachides, de la teneur en eau des arachides et du rapport de l'acide oléique et de l'acide linoléique contenus dans les arachides ;
le calcul du facteur d'évaluation de la qualité associé aux arachides en fonction de la formule *γ* = 30,287+9,995×*a*+0,133×*b*-0,686×*c* ; dans laquelle *γ* est le facteur d'évaluation de la qualité associé aux arachides, *a* est le rapport de l'acide oléique et de l'acide linoléique contenus dans les arachides, *b* est le poids pour cent noyaux des arachides en grammes, et *c* et la teneur en eau des arachides ; et
l'évaluation de l'aptitude à l'utilisation des arachides pour un traitement d'arachides de collation en fonction du facteur d'évaluation de la qualité.

2. Le procédé selon la revendication 1, **caractérisé en ce que** l'évaluation de l'aptitude à l'utilisation des arachides pour un traitement d'arachides de collation en fonction du facteur d'évaluation de la qualité comprend :
si *γ*≤50, les arachides sont inappropriées pour un traitement d'arachides de collation ; sinon, les arachides sont appropriées pour un traitement d'arachides de collation.

3. Le procédé selon la revendication 2, caractérisé, si les arachides sont appropriées pour un traitement d'arachides de collation, en ce qu'il comprend :
si *γ*≥52, les arachides sont appropriées à être utilisées pour un traitement d'arachides de collation ; et
si 52≥*γ*≥50, les arachides sont essentiellement appropriées à être utilisées pour un traitement d'arachides de collation.

4. Procédé selon la revendication 1, **caractérisé en ce que** l'obtention de la teneur en eau des arachides et du rapport de l'acide oléique et de l'acide linoléique contenus dans les arachides comprend :
le chauffage des arachides pendant une période de temps prédéfinie, le calcul de la valeur de différence entre la masse des arachides avant chauffage et de la masse des arachides après chauffage, la valeur de différence étant la teneur en eau des arachides ; et
le décorticage des arachides, la détection de l'acide oléique et de l'acide linoléique contenus dans les arachides avec un analyseur proche infrarouge, et le calcul du rapport de l'acide oléique et de l'acide linoléique contenus dans les arachides.

5. Appareil pour l'évaluation de la qualité d'arachides pour un traitement d'arachides de collation, **caractérisé en ce qu'**il comprend :
un module d'obtention, qui est utilisé pour obtenir la masse des arachides décortiquées à mesurer, et l'obtention du poids pour cent noyaux des arachides, de la teneur en eau des arachides et du rapport de l'acide oléique et de l'acide linoléique contenus dans les arachides ;
un module de calcul, qui est utilisé pour calculer le facteur d'évaluation de la qualité associé aux arachides en fonction de la formule *γ* = 30,287+9,995×*a*+0,133×*b*-0,686×*c* ; dans laquelle *γ* est le facteur d'évaluation de la qualité associé aux arachides, *a* est le rapport de l'acide oléique et de l'acide linoléique contenus dans les arachides, *b* est le poids pour cent noyaux des arachides en grammes, et *c* est la teneur en eau des arachides ; et
un module d'évaluation, qui est utilisé pour évaluer l'aptitude à l'utilisation des arachides pour un traitement d'arachides de collation en fonction du facteur d'évaluation de la qualité.

6. Appareil selon la revendication 5, **caractérisé en ce que** le module d'évaluation est en outre utilisé **en ce que** si *γ*≤50, les arachides sont inappropriées pour un traitement d'arachides de collation ; si *γ*≥52, les arachides sont appropriées à être utilisées pour un traitement d'arachides de collation ; et si 52≥*γ*≥50, les arachides sont essentiellement appropriées à être utilisées pour un traitement d'arachides de collation.

7. Support de stockage lisible par ordinateur non transitoire, **caractérisé en ce que** le support de stockage lisible par ordinateur non transitoire stocke des instructions informatiques permettant à l'ordinateur de réaliser les procédés tels que décrits par l'une quelconque des revendications 1 à 4.
